# DEMANDE DE BREVET EUROPEEN

(11) **EP 1 491 183 A2**
(43) Date de publication de la demande: **29.12.2004**
(21) Numéro de dépôt: 04292061.1
(22) Date de dépôt: 13.08.1998
(51) Int. Cl.: A61K 7/48, A61K 35/78

(54) **Utilisation d'un extrait d'au moins un végétal de la famille des Rosacées**

(30) Priorité: 22.09.1997 FR 9711761
(62) Demande divisionnaire de: 98402056.0
(71) Demandeur: L'OREAL, 75008 Paris (FR)
(72) Inventeur: Breton, Lionel, 78000 Versailles (FR); Pineau, Nathalie, 95240 Cormeilles-en-Parisis (FR)
(74) Mandataire: Bernstein, Claire Jacqueline

(57) **Abrégé**

L'invention a pour objet l'utilisation, à titre de principe actif, dans une composition d'au moins un extrait d'au moins un végétal de la famille des Rosacées en tant qu'antagoniste de substance P. L'invention a en outre pour objet l'utilisation, à titre de principe actif, dudit extrait dans une composition, l'extrait ou la composition étant destinés à traiter les désordres associés à un excès de synthèse et/ou de libération de substance P.

L'invention a également pour objet l'utilisation, à titre de principe actif, dudit extrait dans une composition, l'extrait ou la composition étant destinés à traiter les peaux sensibles.

## Description

L'invention a pour objet l'utilisation, à titre de principe actif, dans une composition d'au moins un extrait d'au moins un végétal de la famille des Rosacées en tant qu'antagoniste de substance P. L'invention a en outre pour objet l'utilisation, à titre de principe actif, dudit extrait dans une composition, l'extrait ou la composition étant destinés à traiter les désordres associés à un excès de synthèse et/ou de libération de substance P.
L'invention a également pour objet l'utilisation, à titre de principe actif, dudit extrait dans une composition, l'extrait ou la composition étant destinés à traiter les peaux sensibles ainsi qu'un procédé de traitement cosmétique pour traiter les peaux sensibles.

Il existe chez les mammifères des polypeptides appartenant à la famille des tachykinines qui induisent sur les fibres musculaires lisses des contractions rapides. Parmi les composés de cette famille, on peut citer la neurokinine β, neurokinine α et la substance P.

La substance P est un élément chimique polypeptidique (undécapeptide), élaboré et libéré par une terminaison nerveuse. La localisation de la substance P est spécifique des neurones, tant dans le système nerveux central que dans les organes à la périphérie. Ainsi, de très nombreux organes ou tissus reçoivent des afférences de neurones à substance P, il s'agit notamment des glandes salivaires, de l'estomac, du pancréas, de l'intestin (dans celui-ci, la distribution de la substance P est superposée au plexus nerveux intrinsèque de Meissner et d'Auerbach), du système cardio-vasculaire, de la glande thyroïde, de la peau, de l'iris et des corps ciliaires, de la vessie et bien évidemment des système nerveux central et périphérique.

De par la distribution ubiquitaire de la substance P, de très nombreux désordres sont associés à un excès de synthèse et / ou de libération de substance P.

La substance P intervient notamment dans la transmission de la douleur et dans des maladies du système nerveux central (par exemple l'anxiété, les psychoses, les neuropathies, les troubles neurodégénératifs de type démence sénile d'Alzheimer, démence des sidéens, maladie de Parkinson, syndrome de Down, syndrome de Korsakoff, scléroses multiples, schizophrénie), dans des maladies respiratoires (telles que par exemple les broncho-pneumonie) et inflammatoires (telles que par exemple la polyarthrite rhumatoïde), dans des syndromes allergiques (tels que par exemple l'asthme, les rhinites allergiques, les pharyngites allergiques, l'urticaire, les dermatites eczémateuses), dans des maladies gastro-intestinales (telles que par exemple les ulcères, les colites, la maladie de Crohn), dans des désordres cutanés (tels que par exemple le psoriasis, les maladies prurigineuses, l'herpès, les photodermatoses, les dermatites atopiques, les dermatites de contact, les lichens, les prurigos, les prurits, les érythèmes en particulier solaire, les piqûres d'insectes), dans des fibroses et autres troubles de la maturation des collagènes (tels que par exemple la sclérodermie), dans des troubles cardio-vasculaires, des troubles vasospastiques (tels que par exemple les migraines, la maladie de Reynaud), dans des désordres immunologiques, dans des troubles du tractus urinaire (tels que par exemple l'incontinence, la cystite), dans des maladies rhumatismales, dans certaines maladies dermatologiques (telles que l'eczéma) et dans les affections ophtalmologiques (telles que par exemple les conjonctivites, les uvéites, les prurits oculaires, les douleurs oculaires, les irritations).

L'utilisation d'antagoniste de substance P est l'une des alternatives thérapeutiques efficaces dans toutes les affections citées ci-dessus.

Par antagoniste de substance P, on entend tout composé susceptible d'inhiber partiellement, voire totalement, l'effet biologique de la substance P.
Particulièrement, pour qu'une substance soit reconnue comme un antagoniste de substance P elle doit induire une réponse pharmacologique cohérente (incluant ou non sa fixation au récepteur de la substance P) notamment dans l'un des tests suivants :
- la substance antagoniste doit diminuer l'extravasation du plasma au travers de la paroi vasculaire induite par la capsaïcine ou par une stimulation nerveuse antidromique, ou bien
- la substance antagoniste doit provoquer une inhibition de la contraction des muscles lisses induites par l'administration de substance P.

A ce jour, des antagonistes de substance P sont utilisés pour traiter les désordres indiqués ci-dessus. A cet effet, on peut se référer aux documents US-A-4472305, US-A-4839465, EP-A-101929, EP-A-333174, EP-A-336230, EP-A-394989, EP-A-443132, EP-A-498069, EP-A-515681, EP-A-517589, WO-A-92/22569, GB-A-2216529, EP-A-360390, EP-A-429366, EP-A-430771, EP-A-499313, EP-A-514273, EP-A-514274, EP-A-514275, EP-A-514276, EP-A-520555, EP-A-528495, EP-A-532456, EP-A-545478, EP-A-558156, WO-A-90/05525, WO-A-90/05729, WO-A-91/18878, WO-A-91/18899, WO-A-92/12151, WO-A-92/15585, WO-A-92/17449, WO-A-92/20676, WO-A-93/00330, WO-A-93/00331, WO-A-93/01159, WO-A-93/01169, WO-A-93/01170, WO-A-93/06099, WO-A-93/09116, EP-A-522808 et WO-A-93/01165.

Cependant aucun des documents ci-dessus n'envisage ni ne suggère qu'un extrait d'au moins un végétal de la famille des Rosacées puisse avoir une activité antagoniste de substance P telle que définie ci-dessus et donc puisse être notamment utilisé à titre de principe actif pour traiter les désordres indiqués ci-dessus.

Les plantes de la famille des Rosacées sont surtout utilisées pour leur propriétés aromatiques et ornementales.
Dans l'art antérieur les végétaux de la famille des Rosacées sont connus dans des compositions pour le traitement de maladies urogénitales (FR 76/36295), dans des compositions cosmétiques éclaircissantes (JP08208451) ou de protection contre les ultraviolets (EP-A-781544), pour la préparation de composés antioxydants (EP-A-94/401669), ou encore pour la préparation de composés antimicrobiens et/ou insecticides pour la protection des plantes (DE4327792).

Mais à la connaissance de la demanderesse l'activité antagoniste de substance P d'au moins un végétal de la famille des Rosacées n'a jamais été décrite.

La demanderesse a découvert qu'un extrait d'au moins un végétal de la famille des Rosacées répond aux caractéristiques définies comme caractérisant un antagoniste de substance P et peut donc être utilisé comme antagoniste de substance P.

L'invention a donc pour objet l'utilisation, à titre de principe actif, dans une composition d'une quantité efficace d'au moins un extrait d'au moins un végétal de la famille des Rosacées en tant qu'antagoniste de substance P.

Par principe actif, on entend toute molécule ou extrait susceptible de modifier ou de moduler le fonctionnement d'au moins un système biologique donné.

L'extrait d'au moins un végétal de la famille des Rosacées peut être tout extrait préparé à partir de tout matériel végétal issu d'au moins un végétal de la famille des Rosacées.

Ainsi, l'extrait d'au moins un végétal de la famille des Rosacées utilisé selon l'invention peut être obtenu à partir de matériel végétal issu de plante entière ou de partie de plante comme les feuilles, les tiges, les fleurs, les pétales, les racines ou encore des cellules indifférenciées.

Par cellules végétales indifférenciées, on entend toute cellule végétale ne présentant aucun des caractères d'une spécialisation particulière et capable de vivre par elle-même et non en dépendance avec d'autres cellules. Ces cellules végétales indifférenciées sont éventuellement aptes, sous l'effet d'une induction, à toute différenciation conforme à leur génome.
Selon la méthode de culture choisie, et en particulier selon le milieu de culture choisi, il est possible d'obtenir, à partir d'un même explant, des cellules végétales indifférenciées présentant des caractères différents.

Préférentiellement selon l'invention on utilise des pétales.

L'extrait d'au moins un végétal de la famille des Rosacées peut être tout extrait préparé à partir de tout matériel végétal issu d'au moins un végétal de la famille des Rosacées cultivé *in vivo* ou issu de culture *in vitro*.

Par culture *in vivo* on entend toute culture de type classique c'est à dire en sol à l'air libre ou en serre, ou encore hors sol.

Par culture *in vitro,* on entend l'ensemble des techniques connues de l'homme du métier qui permet de manière artificielle l'obtention d'un végétal ou d'une partie d'un végétal. La pression de sélection imposée par les conditions physico-chimiques lors de la croissance des cellules végétales *in vitro* permet d'obtenir un matériel végétal standardisé et disponible tout au long de l'année contrairement aux plantes cultivées *in vivo*.

Préférentiellement selon l'invention on utilise un végétal issu de culture *in vivo*.

Toute méthode d'extraction connue de l'homme du métier peut être utilisée pour préparer l'extrait contenu dans la composition selon l'invention.

On peut en particulier citer les extraits aqueux, alcooliques ou utilisant un solvant organique.
Par solvant aqueux on entend tout solvant constitué totalement ou pour part d'eau. On peut citer ainsi l'eau elle-même, les solvant hydroalcooliques en toute proportion ou encore les solvants constitués d'eau et d'un composé comme le propylène glycol en toute proportion.

Parmi les solvants alcooliques on peut citer notamment l'éthanol.

On peut également utiliser un extrait préparé par la méthode décrite dans la demande de brevet français n° 95-02379 déposée par la demanderesse.
Ainsi, dans une première étape on broie le matériel végétal dans une solution aqueuse à froid, dans une deuxième étape les particules en suspension sont éliminées de la solution aqueuse issue de la première étape, et dans une troisième étape on stérilise la solution aqueuse issue de la deuxième étape. Cette solution aqueuse correspond à l'extrait.

D'autre part, la première étape peut avantageusement être remplacée par une opération de congélation simple des tissus végétaux (par exemple à -20°C), suivie d'une extraction aqueuse reprenant les deuxième et troisième étapes ci-dessus décrites.

Quel que soit le mode de préparation utilisé selon l'invention des étapes subséquentes visant à favoriser la conservation et/ou la stabilisation peuvent être ajoutées sans pour cela modifier la nature même de l'extrait. Ainsi, par exemple l'extrait obtenu peut être lyophilisé par toutes méthodes classiques de lyophilisation. On obtient ainsi une poudre qui peut être utilisée directement ou bien mélangée dans un solvant approprié avant utilisation.

Préférentiellement, selon l'invention on utilise un extrait aqueux et encore plus préférentiellement un extrait réalisé avec un solvant composé d'eau et de propylène glycol, comme par exemple l'Herbasol® vendu par la société COSMETOCHEM's.

On a vu préalablement dans le texte des exemples de désordres liés à un excès de synthèse et/ou de libération de substance P.

L'invention a en outre pour objet l'utilisation, à titre de principe actif, dans une composition d'une quantité efficace d'un extrait d'au moins un végétal de la famille des Rosacées, l'extrait ou la composition étant destinés à traiter les désordres associés à un excès de synthèse et/ou de libération de substance P.

Ainsi, selon un aspect particulier, l'invention à pour objet l'utilisation à titre de principe actif, dans une composition d'une quantité efficace d'un extrait d'au moins un végétal de la famille des Rosacées, l'extrait ou la composition étant destinés à traiter les désordres cutanés, l'inflammation, les syndromes allergiques, la douleur, les troubles de la maturation du collagène, les désordres du système nerveux central, les troubles respiratoires, les désordres gastro-intestinaux, les fibroses, les troubles cardio-vasculaires, les troubles vasospastiques, les désordres immunologiques ou encore les troubles du tractus urinaires, les maladies rhumatismales ou certaines affections ophtalmologiques.

Dans le domaine des désordres cutanés, il est connu que certaines peaux sont plus sensibles que d'autres. Toutefois, les symptômes des peaux sensibles étaient jusqu'à présent mal caractérisés et le problème de ces peaux était, de ce fait, mal défini ; personne ne connaissait exactement le processus mis en cause dans la sensibilité de la peau. Certains pensaient qu'une peau sensible était une peau qui réagissait aux produits cosmétiques, d'autres qu'il s'agissait d'une peau qui réagissait à plusieurs facteurs extérieurs, pas forcément liés aux produits cosmétiques. On assimilait également les peaux sensibles à des peaux allergiques.

Des tests ont été mis au point pour cerner les peaux sensibles, par exemple des tests à l'acide lactique et au DMSO qui sont connus pour être des substances irritantes : voir par exemple l'article de K. Lammintausta et coll., Dermatoses, 1988, 36, pages 45-49; et l'article de T. Agner et J. Serup, Clinical and Experimental Dermatology, 1989, 14, pages 214-217.

Du fait de la méconnaissance des caractéristiques des peaux sensibles, il était jusqu'à présent très difficile voire impossible de les traiter. En fait, on les traitait indirectement, par exemple en limitant dans les compositions cosmétiques ou dermatologiques l'emploi de produits à caractère irritant tels que les tensioactifs, les conservateurs ou les parfums ainsi que l'emploi de certains actifs cosmétiques ou dermatologiques.

Après de nombreux tests cliniques, la demanderesse a pu déterminer les symptômes liés aux peaux sensibles. Ces symptômes sont en particulier des signes subjectifs, qui sont essentiellement des sensations dysesthésiques. On entend par sensations dysesthésiques des sensations plus ou moins douloureuses ressenties dans une zone cutanée comme les picotements, fourmillements, démangeaisons ou prurits, brûlures, échauffements, inconforts, tiraillements, etc.

La demanderesse a pu montrer en outre qu'une peau sensible n'était pas une peau allergique. En effet, une peau allergique est une peau qui réagit à un agent extérieur, un allergène, qui déclenche une réaction d'allergie. Il s'agit d'un processus immunologique qui ne se produit que lorsqu'un allergène est présent et qui ne touche que les sujets sensibilisés. La caractéristique essentielle de la peau sensible est selon la demanderesse, au contraire, un mécanisme de réponse à des facteurs extérieurs, qui peut concerner tout individu, même si les individus dits à peau sensible y réagissent plus vite que les autres. Ce mécanisme n'est pas immunologique, il est aspécifique.

La demanderesse a trouvé que les peaux sensibles pouvaient être scindées en deux grandes formes cliniques, les peaux irritables et/ou réactives, et les peaux intolérantes.

Une peau irritable et/ou réactive est une peau qui réagit par un prurit, c'est-à-dire par des démangeaisons, ou par des picotements, à différents facteurs tels que l'environnement, les émotions, les aliments, le vent, les frottements, le rasoir, le savon, les tensioactifs, l'eau dure à forte concentration de calcaire, les variations de température ou la laine. En général, ces signes sont associés à une peau sèche avec ou sans dartres, ou à une peau qui présente un érythème.

Une peau intolérante est une peau qui réagit par des sensations d'échauffement, de tiraillements, de fourmillements et/ou de rougeurs, à différents facteurs tels que l'environnement, les émotions, les aliments et certains produits cosmétiques. En général, ces signes sont également associés à une peau hyperséborrhéique ou acnéique avec ou sans dartres, et à un érythème.

Les cuirs chevelus "sensibles" ont une sémiologie clinique plus univoque : les sensations de prurit et/ou de picotements et/ou d'échauffements sont essentiellement déclenchés par des facteurs locaux tels que frottements, savon, tensioactifs, eau dure à forte concentration de calcaire, shampooings ou lotions. Ces sensations sont aussi parfois déclenchées par des facteurs tels que l'environnement, les émotions et/ou les aliments. Un érythème et une hyperséborrhée du cuir chevelu ainsi qu'un état pelliculaire sont fréquemment associés aux signes précédents.

Par ailleurs, dans certaines régions anatomiques comme les grands plis (régions inguinales, génitale, axillaires, poplitées, anale, sous-mammaires, plis du coude) et les pieds, la peau sensible se traduit par des sensations prurigineuses et/ou des sensations dysesthésiques (échauffement, picotements) liées en particulier à la sueur, aux frottements, à la laine, aux tensioactifs, à certaines préparations cosmétiques, à l'eau dure à forte concentration en calcaire et/ou aux variations de température.

Pour déterminer si une peau est sensible ou non, la demanderesse a également mis au point un test. En effet, après avoir effectué un grand nombre de tests dans le but de définir une peau sensible, elle a trouvé de manière surprenante qu'il existait un lien entre les personnes à peau sensible et celles qui réagissaient à une application topique de capsaïcine.

Le test à la capsaïcine consiste à appliquer sur environ 4 cm² de peau 0,05 ml d'une crème comprenant 0,075 % de capsaïcine et à noter l'apparition de signes subjectifs provoqués par cette application, tels que picotements, brûlures et démangeaisons. Chez les sujets à peaux sensibles, ces signes apparaissent entre 3 et 20 minutes après l'application et sont suivis de l'apparition d'un érythème qui débute à la périphérie de la zone d'application.

Jusqu'à présent, la capsaïcine était utilisée comme médicament, en particulier pour traiter les douleurs du zona. La capsaïcine provoque un relargage des neuropeptides, et en particulier des tachykinines qui proviennent de terminaisons nerveuses de l'épiderme et du derme. La demanderesse a constaté que le schéma physiopathologique commun à tous les états des peaux sensibles était lié à une grande aptitude à libérer des tachykinines et plus particulièrement de la substance P dans la peau. Les manifestations dysesthésiques qui sont provoquées par leur libération sont dites "neurogènes".

Personne jusqu'à ce jour n'avait établi un lien entre la substance P et la peau sensible. Les signes cliniques de la peau sensible sont essentiellement subjectifs : picotements, fourmillements, prurits, tiraillements, échauffements, et ils s'associent parfois à des érythèmes. Ces signes sont dus à des facteurs extérieurs aspécifiques. Les symptômes apparaissent essentiellement localisés au visage, au cou et au cuir chevelu, mais peuvent apparaître aussi sur tout le corps.

Ainsi la demanderesse a découvert qu'une des caractéristiques essentielles des peaux sensibles est liée à la libération de substance P et donc que l'utilisation d'antagonistes de substance P peut permettre d'obtenir un effet préventif et/ou curatif des peaux sensibles.

Pour traiter les peaux sensibles, la demanderesse a donc envisagé d'utiliser des antagonistes de substance P. Elle a en effet constaté de manière surprenante que l'incorporation d'un antagoniste de substance P dans une composition destinée à un usage topique permet d'éviter l'irritation et/ou les sensations dysesthésiques et/ou les prurits de la peau.

L'invention concerne donc plus particulièrement l'utilisation, à titre de principe actif, dans une composition d'une quantité efficace d'au moins un extrait d'au moins un végétal de la famille des Rosacées, l'extrait ou la composition étant destinés à traiter les peaux sensibles.

La présente invention a encore pour objet l'utilisation, à titre de principe actif, dans une composition d'une quantité efficace d'au moins un extrait d'au moins un végétal de la famille des Rosacées, l'extrait ou la composition étant destinés à prévenir et/ou à lutter contre les irritations cutanées et/ou les dartres et/ou les érythèmes et/ou les sensations d'échauffement et/ou de dysesthésie et/ou les prurits de la peau et/ou les muqueuses.

La famille des Rosacées comprend 27 genres parmi lesquels on peut citer à titre d'exemple les genres Agrimonia, Amygdalus, Armeniaca, Cerasus, Malus, Mespilus, Persica, Pirus, Prunus, Rosa, Rubus.

Ainsi, l'extrait de Rosacées de l'invention est un extrait préparé à partir de matériel issu d'au moins un végétal appartenant à un genre choisi parmi Agrimonia, Amygdalus, Armeniaca, Cerasus, Malus, Mespilus, Persica, Pirus, Prunus, Rosa, Rubus.

Préférentiellement selon l'invention le végétal appartient au genre Rosa.

Le genre Rosa comporte plus de 1000 espèces parmi lesquelles on peut citer *Rosa alba, Rosa alpina, Rosa canina, Rosa cinnamonea, Rosa gallica, Rosa repens, Rosa rubrifolia, Rosa rubiginosa, Rosa sempervirens, Rosa spinosissima, Rosa stylosa, Rosa tomentosa ou encore Rosa villosa.*

Ainsi, l'extrait de végétal du genre Rosa de l'invention est un extrait préparé à partir de matériel issu d'au moins un végétal appartenant à une espèce choisie parmi *Rosa alba, Rosa alpina, Rosa canina, Rosa cinnamonea, Rosa gallica, Rosa repens, Rosa rubrifolia, Rosa rubiginosa, Rosa sempervirens, Rosa spinosissima, Rosa stylosa, Rosa tomentosa, Rosa villosa.*

Préférentiellement selon l'invention le végétal appartient à l'espèce *Rosa gallica.*

Selon l'invention, la quantité d'extrait d'au moins un végétal de la famille des Rosacées utilisée dans la composition est bien entendu fonction de l'effet recherché et peut donc varier dans une large mesure.

Pour donner un ordre de grandeur, selon l'invention, l'extrait peut être utilisé en une quantité représentant de 0,01% à 30% du poids total de la composition et préférentiellement en une quantité représentant de 0,1% à 20% du poids total de la composition.

On a vu précédemment dans le texte ce qu'est une peau irritable. L'irritation cutanée peut avoir de multiples causes. Ce peut être des causes intrinsèques, liés au dérèglement des mécanismes physiologiques conduisant à une peau normale. Mais ce peut être également des causes extrinsèques comme des composés irritants qui viendraient en contact avec la peau.

Ainsi, la présente invention a en outre pour objet un procédé de traitement cosmétique en vue de diminuer l'irritation cutanée, caractérisé par le fait que l'on utilise par application sur la peau, sur les cheveux, et/ou sur les muqueuses, une composition cosmétique comprenant au moins un extrait d'au moins un végétal de la famille des Rosacées dans un milieu cosmétiquement acceptable.

Par milieu cosmétiquement acceptable, on entend compatible avec la peau, le cuir chevelu, les muqueuses, les ongles, et les cheveux.

Le procédé de traitement cosmétique de l'invention peut être mis en oeuvre notamment en appliquant les compositions hygiéniques ou cosmétiques telles que définies ci-dessus, selon la technique d'utilisation habituelle de ces compositions. Par exemple : application de crèmes, de gels, de sérums, de lotions, de laits de démaquillage ou de compositions anti-solaires sur la peau ou sur les cheveux secs, application d'une lotion pour cheveux sur cheveux mouillés, de shampooings, ou encore application de dentifrice sur les gencives.

De façon avantageuse, selon l'invention au moins un extrait d'au moins un végétal de la famille des Rosacées peut être associé à des produits à effet irritant utilisés couramment dans le domaine cosmétique ou pharmaceutique, produits qui sont parfois des actifs cosmétiques ou pharmaceutiques. La présence d'un antagoniste de substance P sous la forme d'au moins un extrait d'au moins un végétal de la famille des Rosacées dans une composition cosmétique ou pharmaceutique comprenant un produit ayant un effet irritant permet d'atténuer fortement, voire de supprimer cet effet irritant.

Cela permet en outre d'augmenter la quantité de principe actif à effet irritant par rapport à la quantité de principe actif normalement utilisée, en vue d'une efficacité améliorée.

L'invention concerne plus particulièrement une composition, caractérisée en ce qu'elle comprend, dans un milieu physiologiquement acceptable, au moins un produit à effet irritant et au moins un extrait d'au moins un végétal de la famille des Rosacées.

Par milieu physiologiquement acceptable, on entend compatible avec la peau, le cuir chevelu, les muqueuses, les ongles, et les cheveux.

Dans cette forme de l'invention l'extrait d'au moins un végétal de la famille des Rosacées est un extrait comme décrit précédement dans le texte.

Comme produits à effet irritant, on peut citer par exemple les tensioactifs (ioniques ou non-ioniques), les conservateurs, les solvants organiques ou les actifs comme les α-hydroxy-acides (acide citrique, malique, glycolique, tartrique, mandélique, lactique), les β-hydroxy-acides (l'acide salicylique et ses dérivés), les α-céto-acides, les β-céto-acides, les rétinoïdes (rétinol, rétinal, acide rétinoïque), les anthralines (dioxyanthranol), les anthranoïdes, les peroxydes (notamment de benzoyle), le minoxidil, les sels de lithium, les antimétabolites, la vitamine D et ses dérivés, les teintures ou colorants capillaires (paraphénylènediamine et ses dérivés, les aminophénols), les solutions alcooliques parfumantes (parfums, eaux de toilette, après rasage, déodorants), les agents antitranspirants (certains sels d'aluminium), les actifs dépilatoires ou de permanentes (thiols), les actifs dépigmentants (hydroquinone).

L'emploi d'antagoniste de substance P permet notamment de multiplier de 2 à 10 fois la quantité de principe actif à effet irritant par rapport à l'état de la technique, sans ressentir tous les inconforts mentionnés ci-dessus. Ainsi, on peut utiliser les hydroxyacides jusqu'à 50 % du poids de la composition ou les rétinoïdes jusqu'à 5 %, en diminuant notablement leur caractère irritant.

On sait par ailleurs qu'il existe au niveau cutané de nombreux phénomènes d'intolérance dont les symptômes sont en particulier des signes subjectifs, qui sont essentiellement des sensations dysesthésiques. On entend par sensations dysesthésiques des sensations plus ou moins douloureuses ressenties dans une zone cutanée comme les picotements, fourmillements, démangeaisons ou prurits, brûlures, échauffements, inconforts, tiraillements, etc.

Ces phénomènes peuvent être la conséquence d'événements multiples dont les plus banaux seront qualifiés d'irritation ou d'inflammation, mais dont certains seront dus à des causes physiologiques, comme les peaux sensibles, voire même pathologiques comme par exemple, l'allergie.

Mais, la peau sensible peut également réagir par des sensations d'échauffement, de tiraillements, de fourmillements et/ou de rougeurs à différents facteurs tels que l'environnement, les émotions ou les aliments. En général, ces signes sont associés à une peau hyperséborrhéique ou acnéique, avec ou sans dartres. Là encore, ces signes sont souvent associés à un érythème.

Ces phénomènes peuvent être généralisés à l'ensemble du corps, mais la plupart du temps ils peuvent avoir des localisations bien définies telles que par exemple le cuir chevelu, le visage, les plis cutanés, etc.

L'ensemble de ces phénomènes d'intolérance est toujours lié à un processus inflammatoire classique, et plus particulièrement à une réaction inflammatoire de type neurogène puisqu'elle fait intervenir les fibres nerveuses cutanées.

Une peau allergique est une peau qui réagit à un agent extérieur, un allergène, qui déclenche une réaction d'allergie. Il s'agit alors d'un processus spécifiquement immunologique qui ne se produit que lorsqu'un allergène est présent et qui ne touche que les sujets sensibilisés. Par contre, la résultante finale d'une réaction allergique se traduit également par une réaction inflammatoire aiguë associé généralement à un oedème.

Quelque soit le phénomène envisagé, il existe un point commun à tous ces mécanismes qui se traduit par une réaction inflammatoire dont la facette terminale se mesure par la libération par les cellules mastocytaires de la peau d'au moins un médiateur de l'inflammation tels que l'histamine, la sérotonine, l'héparine, les leukotriènes, les prostaglandines, les cytokines, le monoxyde d'azote ou des espèces oxygénées réactives.

Dans certains cas, comme par exemple les peaux sensibles, l'ensemble du mécanisme est également sous le contrôle des terminaisons nerveuses sensitives qui libèrent des neuropeptides, notamment la substance P et le peptide dérivé de la calcitonine (connu sous le nom Calcitonine Gene Related Peptide ou CGRP).

Le but recherché par la présente invention est d'obtenir un effet bénéfique le plus étendu possible dans le traitement de toutes ces affections cutanées et donc de proposer une composition qui agit sur plusieurs composantes de ces affections.

Ainsi, selon un autre aspect la présente invention a pour objet une composition, caractérisée en ce qu'elle comprend, dans un milieu physiologiquement acceptable, au moins un extrait d'au moins un végétal de la famille des Rosacées et au moins un composé diminuant la synthèse, la libération et/ou l'activité d'au moins un médiateur de l'inflammation.

Préférentiellement, l'extrait d'au moins un végétal de la famille des Rosacées est un extrait comme décrit précédement dans le texte.

Le composé diminuant la synthèse, la libération et/ou l'activité d'au moins un médiateur de l'inflammation est de préférence choisi parmi les antagonistes de substance P et/ou de CGRP, les inhibiteurs de NO-synthase, les antagonistes de bradykinine, les antagonistes de cytokines, les antagonistes d'histamine, les antagonistes du facteur de nécrose tumorale de type α (TNFα).

Préférentiellement, on utilise des antagonistes réceptoriels.

Par exemple, selon l'invention, on peut utiliser un ou plusieurs antagonistes de substance P choisis parmi les peptides, les composés non peptidiques comme ceux comprenant au moins un hétérocycle, les composés azotés comprenant au moins un cycle benzénique, les sels de cations monovalents, divalents et trivalents, les eaux thermales, les extraits végétaux, les extraits de micro-organismes, particulièrement de bactéries, et leurs mélanges.

On peut utiliser dans l'invention par exemple comme peptide antagoniste de substance P le sendide et le spantide II.

Le sendide correspond à la formule :

Tyr D-Phe Phe D-His Leu Met NH₂

dans laquelle :
Tyr représente la tyrosine,
D-Phe représente la D-phénylalanine,
Phe représente la phénylalanine,
D-His représente la D-histidine,
Leu représente la leucine,
Met représente la méthionine.

Le spantide II correspond à la formule :

D-NicLys Pro 3-Pal Pro D-Cl₂Phe Asn D-Trp Phe D-Trp Leu Nle NH₂

dans laquelle :
D-NicLys représente le nicotinate de D-lysine,
Pro représente la proline,
3-Pal représente la 3-pyridyl-alanine,
D-Cl₂Phe représente la D-dichlorophénylalanine,
Asn représente l'asparagine,
D-Trp représente le D-tryptophane,
Phe représente la phénylalanine,
Leu représente la leucine,
Nle représente la nor-leucine.

On peut également utiliser dans l'invention, comme peptide antagoniste de substance P, les peptides décrits dans les documents US-A-4472305, US-A-4839465, EP-A-101929, EP-A-333174, EP-A-336230, EP-A-394989, EP-A-443132, EP-A-498069, EP-A-515681, EP-A-517589, WO-A-92/22569 et GB-A-2216529.

Les antagonistes de substance P non peptidiques utilisables dans l'invention sont notamment des composés comprenant un hétéroatome lié directement ou indirectement à un cycle benzénique ou contenu dans un hétérocycle. En particulier cet hétéroatome est un atome d'oxygène, d'azote ou de soufre.

Comme composé hétérocyclique, on peut notamment utiliser dans l'invention ceux décrits dans les documents suivants : EP-A-360390, EP-A-429366, EP-A-430771, EP-A-499313, EP-A-514273, EP-A-514274, EP-A-514275, EP-A-514276, EP-A-520555, EP-A-528495, EP-A-532456, EP-A-545478, EP-A-558156, WO-A-90/05525, WO-A-90/05729, WO-A-91/18878, WO-A-91/18899, WO-A-92/12151, WO-A-92/15585, WO-A-92/17449, WO-A-92/20676, WO-A-93/00330, WO-A-93/00331, WO-A-93/01159, WO-A-93/01169, WO-A-93/01170, WO-A-93/06099, WO-A-93/09116.

En particulier, le composé comprenant au moins un hétérocycle azoté est un dérivé de 2-tricyclyl-2-amino-éthane, un dérivé de spirolactame, un dérivé de quinuclidine, un dérivé azacyclique, un dérivé d'aminopyrrolidine, un dérivé de pipéridine, un aminoazahétérocycle ou un dérivé d'isoindole.

Comme autres composés hétérocycliques, on peut citer les composés hétérocycliques oxygénés ou soufrés tels que les dérivés du furanne, les dérivés du benzofuranne, les dérivés du thiophène et les dérivés du benzothiophène, comportant éventuellement des substituants azotés, tels que les composés hétérocycliques décrits dans les documents US-A-4931459, US-A-4910317 et EP-A-299457, et plus spécialement les alcoxy- et/ou aryloxy- tétrazolyl-benzofuranne-carboxamides ou les alcoxy- et/ou aryloxy- tétrazolyl-benzothiophène-carboxamides.

Comme composés comportant un atome d'azote lié directement ou indirectement à un noyau benzénique, on peut citer ceux décrits dans les documents suivants : EP-A-522808 et WO-A-93/01165 et WO-A-93/10073.

Les sels de cations utilisables dans l'invention sont notamment les sels de strontium, de magnésium, de lanthanides de numéro atomique allant de 57 à 71, de cobalt, de nickel, de manganèse, de baryum, d'yttrium, de cuivre, d'étain, de rubidium, de lithium et de zinc.

Ces sels peuvent être par exemple des carbonates, des salicylates, des bicarbonates, des sulfates, des glycérophosphates, des borates, des chlorures, des nitrates, des acétates, des hydroxydes, des persulfates ainsi que des sels d'α-hydroxyacides (citrates, tartrates, lactates, malates) ou d'acides de fruits, ou encore des sels d'acides aminés (aspartate, arginate, glycocholate, fumarate) ou des sels d'acides gras (palmitate, oléate, caséinate, béhénate). De préférence, le sel est choisi parmi le nitrate de strontium, de manganèse, d'yttrium ou de magnésium, le borate de strontium, de manganèse, d'yttrium ou de magnésium, le chlorure de strontium, de manganèse ou de magnésium, le sulfate de magnésium, de manganèse ou de strontium. Encore plus préférentiellement, ces sels sont le chlorure ou le nitrate de strontium.

Parmi les eaux thermales utilisables selon l'invention on peut citer plus particulièrement les eaux thermales du bassin de Vichy, comme celles provenant des sources Célestins, Chomel, Grande-Grille, Hôpital, Lucas et Parc.
Préférentiellement, selon l'invention, on utilise l'eau de la source Lucas.

Parmi les extrait végétaux utilisables selon l'invention on peut citer particulièrement les extraits préparés à partir de végétaux de la famille des Iridacées, tels que ceux décrits dans la demande de brevet européen EP-A-0765668.
Parmi les extraits de micro-organismes utilisables selon l'invention on peut citer particulièrement les extraits préparés à partir de bactéries filamenteuses non photosynthétiques, tels que ceux décrits dans la demande de brevet européen EP-A-0761204.

Les antagonistes de substance P peuvent être utilisés seuls ou en mélange.

Par antagoniste du CGRP, on entend tout composé susceptible d'inhiber partiellement, voire totalement, l'effet biologique du CGRP.
Particulièrement, pour qu'une substance soit reconnue comme un antagoniste de CGRP elle doit induire une réponse pharmacologique cohérente (incluant ou non sa fixation au récepteur du CGRP) notamment dans l'un des tests suivants :
+ la substance antagoniste doit diminuer la vasodilatation induite par la capsaïcine et/ou par une stimulation électrique antidromique (appliquée sur un nerf afférent) et/ou
+ la substance antagoniste doit provoquer une inhibition de la libération de CGRP par les fibres nerveuses sensitives et/ou
+ la substance antagoniste doit provoquer une inhibition de la contraction du muscle lisse du vas deferens induite par le CGRP.

Parmi les antagonistes du CGRP connus, on peut citer par exemple le CGRP 8-37 (séquence des acides aminés 8 à 37 de la partie N-terminale du CGRP), ou encore les anticorps anti-CGRP.
On peut encore citer les extraits végétaux préparés à partir de végétaux de la famille des Iridacées tels que ceux décrits dans la demande de brevet européen EP-A-0765668.

Les antagonistes de CGRP peuvent être utilisés seuls ou en mélange.

Le terme NO-synthase recouvre en fait une famille d'enzymes qui, de façon spécifique, assurent la catalyse enzymatique de la L-arginine en citrulline, catalyse au cours de laquelle est produit un médiateur gazeux aux multiples fonctions, le monoxyde d'azote ou NO. Le monoxyde d'azote possède de par sa structure un électron supplémentaire le rendant extrêmement réactif chimiquement. Il est notoire que de tels composés sont nocifs et l'on cherche à limiter au mieux leur production. C'est ainsi que dans le cas du monoxyde d'azote les inhibiteurs de NO-synthase ont été largement étudiés.

Ainsi, selon l'invention, les inhibiteurs de NO-synthase sont des produits qui permettent *in situ* sur l'homme d'inhiber partiellement, voire totalement, la synthèse de monoxyde d'azote (NO).
Ce sont donc des composés choisis parmi les composés inhibant la synthèse et/ou accélérant le catabolisme de la NO-synthase, les composés neutralisant la NO-synthase ou les composés intervenant en diminuant le signal transduit par la NO-synthase.

Ainsi, l'inhibiteur de la NO-synthase peut être choisi parmi des peptides, synthétiques ou naturels, éventuellement modifiés, des molécules chimiques, synthétiques ou naturelles, des acides nucléiques antisens, des ribozymes, des anticorps anti-NO-synthase.

Parmi ces inhibiteurs de la NO-synthase, on peut citer notamment la N^{G}-monométhyl-L-arginine (L-NMMA), la N^{G}-nitro-L-arginine, l'ester méthylé de la N^{G}-nitro-L-arginine, le chlorure de diphénylèneiodonium, la 7-nitroindazole, la N(5)-(1-iminoéthyl)-L-ornithine, la N^{G},N^{G}-diméthyl-L-arginine, la N^{G},N^{G}-diméthyl-arginine, le 2-(4-carboxyphényl)-4,4,5,5-tetraméthylimidazoline-1-oxy-3-oxyde, l'aminoguanidine, la canavanine, l'ebselen et l'hormone stimulatrice des mélanocytes de type α.

Parmi les inhibiteurs de la NO-synthase, on utilise préférentiellement la N^{G}-monométhyl-L-arginine et l'hormone stimulatrice des mélanocytes de type α. Les inhibiteurs de la NO-synthase peuvent être utilisés seuls ou en mélange.

La bradykinine est un peptide d'origine plasmatique libéré à partir d'un précurseur kininogène par une protéase plasmatique du nom de kallikreine (EC 3.4.21.24). Ce nanopeptide est un des médiateurs clés de l'inflammation et possède des propriétés mitogènes. Les récepteurs pour cette kinine se divisent en deux principaux sous types, B1 et B2. La bradykinine agit notamment, sur le récepteur B2 et provoque la stimulation de nombreux systèmes de production de seconds messagers dont l'inositol-triphosphates (ip3), connu pour provoquer la libération de calcium à partir des sites de stockages intracellulaires dans les cellules dont le kératinocyte. La bradykinine induit également la phosphorylation des résidus tyrosine par l'intermédiaire d'une activation du récepteur B2, la dépolarisation ou l'hyperpolarisation de la membrane cellulaire ainsi que l'activation du métabolisme de l'acide arachidonique.

Par antagoniste de la bradykinine, on entend tout composé susceptible d'inhiber partiellement, voire totalement, l'effet biologique de la bradykinine.

Particulièrement, pour qu'une substance soit reconnue comme un antagoniste de la bradykinine elle doit induire une réponse pharmacologique cohérente incluant ou non sa fixation au récepteur de la bradykinine.

Ainsi, entre dans cette définition tout composé qui peut interférer avec les effets de la bradykinine par sa fixation au récepteur de celle-ci (B1 ou B2) et/ou tout composé qui indépendamment de la fixation au(x) récepteur(s) induit par un mécanisme quelconque un effet contraire à celui connu de la bradykinine (par exemple interférant avec la synthèse de la bradykinine).

Parmi les antagonistes de la bradykinine, on préfère utiliser des composés inhibant la synthèse et/ou accélérant le catabolisme de la bradykinine, des composés neutralisant la bradykinine, des composés bloquant les récepteurs de la bradykinine tels que ceux qui interférent avec les effets de la bradykinine par leur fixation au récepteur de celle-ci (B1 ou B2), des composés inhibant la synthèse des récepteurs de la bradykinine ou des composés intervenant en diminuant le signal transduit par la bradykinine. Ces composés peuvent être d'origine naturelle ou synthétique.

Parmi les antagonistes de la bradykinine, on peut citer plus particulièrement des peptides, synthétiques ou naturels, éventuellement modifiés, comme la D-Arg, [Hyp3, D-Phe7]-bradykinin (NPC567), la [Thi 5, 8, D-Phe7]-bradykinin, la D-Arg, [Hyp3, Thi5,8, D-Phe7]-bradykinin, la N-α-adamantaneacetyl-D-Arg, [Hyp3, Thi5,8, D-Phe7]-bradykinin, la des-Arg9, [Leu8]-bradykinin (tous vendus par la société Sigma) ou encore les composés cités dans les brevets WO 95/08566, WO 95/07294, EP 0623350, EP 0622361, WO 94/11021, EP 0596406, WO 94/06453, WO 94/09001, EP 0578521, EP 0564972, EP 0552106, WO 93/11789, US 5216165, US 5212182, WO 92/17201, EP 0496369, EP 0472220, EP 0455133, WO 91/09055, WO 91/02746, EP 0413277, EP 0370453, EP 0359310, WO 90/03980, WO 89/09231, WO 89/09230, WO 89/01780, EP 0334244, EP 0596406, WO 86/07263 ou la P-guanidobenzoyl, [Hyp3,Thi5,D-Tic7,Oic8]-bradyklnin (S 16118) (Feletou M & al., Pharmacol. Exp. Ther., June 1995, 273, 1078-84), la D-Arg, [Hyp3, Thi5, D-Tic7,Oic8]-bradykinine (HOE 140) (Feletou M & al., Eur. J. Pharmacol. 1995, 274, 57-64), la D-Arg, [Hyp3, D-Hype (trans-propyl) 7, Oic 8]-bradykinin (NPC 17731) ( Herzig M.C.S. and Leeb-Lundberg L.M.F., J. Biol. Chem. 1995, 270, 20591-20598) ou ceux cités dans Bradykinin Antagonists : development and applications (Stewart J.M., Biopolymers, 1995, 37, 143-155), ou encore des molécules chimiques, synthétiques ou naturelles, comme par exemple celles décrites dans Salvino et coll. J. Med. Chem., 1993, 36, 2583-2584.

On peut également utiliser selon l'invention des acides nucléiques antisens ou des ribozymes ayant pour but de sélectivement inhiber la synthèse de la bradykinine. Ces acides nucléiques antisens sont connus de l'homme du métier. Ils peuvent agir de différentes manières sur l'ADN ou sur l'ARN messager codant pour la bradykinine, notamment par blocage de la fixation ou de la progression des ribosomes le long de l'ARN messager, par clivage de l'ARN messager par la RNase H, ou en empêchant le transport de l'ARN messager du noyau vers le cytoplasme ou encore en empêchant la maturation de l'ARN messager.

On peut encore utiliser selon l'invention des anticorps anti-bradykinine ou des récepteurs solubles de la bradykinine, des anticorps anti-récepteurs de la bradykinine ou des antagonistes des récepteurs de la bradykinine.

Préférentiellement, selon l'invention on utilise un composé qui interfère avec les effets de la bradykinine par sa fixation au récepteur de celle-ci (B1 ou B2), préférentiellement au récepteur B2.

Encore plus préférentiellement, on utilise selon l'invention un antagoniste de la bradykinine choisi parmi :
la D-Arg, [Hyp3, D-Phe7]-bradykinin (NPC567),
la [Thi 5, 8, D-Phe7]-bradykinin, la D-Arg, [Hyp3, Thi5,8, D-Phe7]-bradykinin,
la N-α-adamantaneacetyl-D-Arg, [Hyp3, Thi5,8, D-Phe7]-bradykinin,
la des-Arg9, [Leu8]-bradykinin,
la P-guanidobenzoyl, [Hyp3,Thi5,D-Tic7,Oic8]-bradyklnin (S 16118),
la D-Arg, [Hyp3, Thi5, D-Tic7,Oic8]-bradykinine (HOE 140),
la D-Arg, [Hyp3, D-Hype (trans-propyl) 7, Oic 8]-bradykinin (NPC 17731).

Le peptide modifié préférentiellement utilisé selon l'invention est la D-Arg, [Hyp3, Thi5, D-Tic7,Oic8]-bradykinine (HOE 140).

Les antagonistes de bradykinine peuvent être utilisés seuls ou en mélange.

On sait, par ailleurs, que la substance P libérée par les terminaisons sensitives épidermiques induit une cascade d'événements biochimiques dont les premières étapes se situent au niveau des mastocytes. La fixation de la substance P sur les récepteurs mastocytaires induit une libération de nombreux médiateurs pro-inflammatoires parmi lesquels l'histamine, les cytokines comme l'interleukine 1 (IL1), l'interleukine 6 (IL6), l'interleukine 8 (IL8) et le facteur de nécrose tumorale de type α (Tumor Necrosis Factor α (TNF-α)).

On entend par antagonistes d'histamine, de cytokines et/ou de TNF-α, toutes substances susceptibles d'inhiber la libération et/ou la synthèse et/ou la fixation réceptorielle respectivement d'histamine, de cytokines et/ou de TNF-α.

Les antagonistes inhibant la fixation réceptorielle de l'histamine sont des agents spécifiques du récepteur de type 1 de l'histamine (H1).

Pour qu'une substance soit reconnue comme un antagoniste réceptoriel d'histamine, de cytokines ou de TNF-α, elle doit répondre à l'une des caractéristiques suivantes :
- avoir une affinité pour les récepteurs spécifiques de ces composés ;
- avoir une activité pharmacologique antagoniste réceptoriel d'histamine, de cytokines ou de TNF-α, c'est-à-dire induire une réponse pharmacologique cohérente dans l'un des tests suivants :
   + pour les antagonistes réceptoriels d'histamine : une inhibition de la contraction des muscles lisses induite par l'administration d'histamine ;
   + pour les antagonistes réceptoriels de cytokines: inhibition de l'adhésion de macrophages induite par les cytokines sur les cellules endothéliales ou inhibition de la libération d'anions superoxydes induite par les cytokines sur les neutrophiles ;
   + pour les antagonistes réceptoriels de TNF-α : inhibition de l'adhésion de macrophages induite par TNF-α sur les cellules endothéliales ou inhibition de la libération d'anions superoxydes induite par TNF-α sur les neutrophiles ou inhibition de l'activité mitogène du TNF-α sur les fibroblastes du derme.

Pour qu'une substance soit reconnue comme un antagoniste de la libération et/ou de la synthèse d'histamine, de cytokines ou de TNF-α, elle doit répondre à l'une des caractéristiques suivantes :
- inhibition de la libération d'histamine par des mastocytes stimulés par le composé 48/80 ou stimulés par un ionophore calcique (A23 187)
- inhibition de la libération de cytokines ou de TNF-α par des monocytes (cellules U937) différenciés par un ester de phorbol (PMA).

Les antagonistes réceptoriels d'histamine H1 utilisables dans l'invention sont ceux classiquement utilisés dans les traitements des états allergiques et anaphylactiques ainsi que ceux pour lutter contre le mal des transports. Ces composés peuvent être par exemple des dérivés de la diéthylène diamine comme la Cinnarizine, la cyclizine ; des dérivés de l'aminopropane comme la dexchloro-phéniramine, la triprolidine ; des dérivés de phénothiazine comme la prométhazine, l'alimémazine ; ainsi que les composés cités pages 116 à 118 du livre Joseph R. Prous, The Year's Drug News, Therapeutic Targets, édition 1994, Prous Science Publishers comme la cétirizine HCl, l'ébastine, la loratadine, la sétastine HCl.

Les inhibiteurs de libération de l'histamine sont notamment des composés hétérocycliques oxygénés ou soufrés tels que les dérivés du furanne, les dérivés du benzofuranne, les dérivés du thiophène et les dérivés du benzothiophène, comportant éventuellement des substituants azotés, tels que ceux décrits dans les documents US-A-4931459, US-A-4910317 et EP-A-299457, et plus spécialement les alcoxy- et/ou aryloxy- tétrazol-yl-benzofuranne-carboxamides ou les alcoxy- et/ou aryloxy- tétrazol-yl-benzothiophène-carboxamides. A titre d'exemple, on peut citer le 5-méthoxy-3-phénoxy-N-1H-tétrazol-5-yl-benzothiophène-2-carboxamide, le 5-méthoxy-3-(1-méthyléthoxy)-N-1H-tétrazol-5-yl-benzothiophène-2-carboxamide, le 6-méthoxy-3-(1-méthyléthoxy)-N-1H-tétrazol-5-yl-benzothiophène-2-carboxamide, le 5-méthoxy-3-(1-méthyléthyl)-N-1 H-tétrazol-5-yl-benzothiophène-2-carboxamide, le 3-benzyloxy-5-méthoxy-N-1H-tétrazol-5-yl-benzothiophène-2-carboxamide, et le 5-méthoxy-3-phénoxy-N-1H-tétrazof-5-yl-benzothiophène-2-carboxamide.

Parmi les antagonistes de cytokines, on citera par exemple un antagoniste de la libération de l'interleukine 1 utilisable dans l'invention qui peut être l'auranofine, le SKF-105809 ou le tripeptide Lys-Pro-Val ou encore un antagoniste de la synthèse d'interleukine 1 qui peut être la lactoférrine.

Les antagonistes réceptoriels de TNF-α et les inhibiteurs de la libération et/ou de la synthèse de TNF-α utilisables dans l'invention sont en particulier la lisophyline, l'A802715, la sulfasalazine.

Les antagonistes d'histamine, de cytokines et de TNF-a peuvent être synthétisés ou extraits de produits naturels (végétaux ou animaux).

Les antagonistes d'histamine, de cytokines et de TNF-a peuvent être utilisés selon l'invention, séparément ou associés, seuls ou sous forme de mélange.

La quantité de composé diminuant la synthèse, la libération et/ou l'activité d'au moins un médiateur de l'inflammation contenue dans la composition de l'invention est bien entendu fonction de l'effet recherché et peut donc varier dans une large mesure.

Pour donner un ordre de grandeur, la composition de l'invention peut contenir un composé diminuant la synthèse, la libération et/ou l'activité d'au moins un médiateur de l'inflammation en une quantité représentant de 0,001% à 10% du poids total de la composition et préférentiellement en une quantité représentant de 0,01% à 5% du poids total de la composition.

Quelque soit la forme de la composition selon l'invention, l'extrait de Rosacées utilisé est un extrait tel que décrit précédemment dans le texte

Bien entendu selon l'invention, la composition peut être une composition à usage cosmétique ou pharmaceutique. Préférentiellement, selon l'invention la composition est une composition à usage cosmétique.

Quelque soit la forme de la composition selon l'invention dans laquelle au moins un extrait d'au moins un végétal de la famille des Rosacées est utilisé, celle-ci peut être ingérée, injectée ou appliquée sur la peau (sur toute zone cutanée du corps), les cheveux, les ongles ou les muqueuses (buccale, jugale, gingivale, génitale, conjonctive). Selon le mode d'administration, la composition selon l'invention peut se présenter sous toutes les formes galéniques normalement utilisées.

Pour une application topique sur la peau, la composition peut avoir la forme notamment de solution aqueuse ou huileuse ou de dispersion du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle du type crème ou gel aqueux ou anhydres, ou encore de microcapsules ou microparticules, ou de dispersions vésiculaires de type ionique et/ou non ionique. Ces compositions sont préparées selon les méthodes usuelles.

Elles peuvent être également utilisées pour les cheveux sous forme de solutions aqueuses, alcooliques ou hydroalcooliques, ou sous forme de crèmes, de gels, d'émulsions, de mousses ou encore sous forme de compositions pour aérosol comprenant également un agent propulseur sous pression.

Pour l'injection, la composition peut se présenter sous forme de lotion aqueuse, huileuse ou sous forme de sérum. Pour les yeux, elle peut se présenter sous forme de gouttes et pour l'ingestion, elle peut se présenter sous forme de capsules, de granulés de sirops ou de comprimés.

Les quantités des différents constituants des compositions selon l'invention sont celles classiquement utilisées dans les domaines considérés.

Ces compositions constituent notamment des crèmes de nettoyage, de protection, de traitement ou de soin pour le visage, pour les mains, pour les pieds, pour les grands plis anatomiques ou pour le corps, (par exemple crèmes de jour, crèmes de nuit, crèmes démaquillantes, crèmes de fond de teint, crèmes anti-solaires), des fonds de teint fluides, des laits de démaquillage, des laits corporels de protection ou de soin, des laits anti-solaires, des lotions, gels ou mousses pour le soin de la peau, comme des lotions de nettoyage, des lotions anti-solaires, des lotions de bronzage artificiel, des compositions pour le bain, des compositions désodorisantes comprenant un agent bactéricide, des gels ou lotions après-rasage, des crèmes épilatoires, des compositions contre les piqûres d'insectes, des compositions anti-douleur, des compositions pour traiter certaines maladies de la peau comme l'eczéma, la rosasée, le psoriasis, les lichens, les prurits sévères.

Les compositions selon l'invention peuvent également consister en des préparations solides constituant des savons ou des pains de nettoyage.

Les compositions peuvent aussi être conditionnées sous forme de composition pour aérosol comprenant également un agent propulseur sous pression.

La composition selon l'invention peut aussi être une composition pour soins capillaires, et notamment un shampooing, une lotion de mise en plis, une lotion traitante, une crème ou un gel coiffant, une composition de teintures (notamment teintures d'oxydation) éventuellement sous forme de shampooings colorants, des lotions restructurantes pour les cheveux, une composition de permanente (notamment une composition pour le premier temps d'une permanente), une lotion ou un gel antichute, un shampooing antiparasitaire, etc.

La composition peut aussi être à usage bucco-dentaire, par exemple une pâte dentifrice. Dans ce cas, la composition peut contenir des adjuvants et additifs usuels pour les compositions à usage buccal et notamment des agents tensioactifs, des agents épaississants, des agents humectants, des agents de polissage tels que la silice, divers ingrédients actifs comme les fluorures, en particulier le fluorure de sodium, et éventuellement des agents édulcorants comme le saccharinate de sodium.
Lorsque la composition est une émulsion, la proportion de la phase grasse peut aller de 5 % à 80 % en poids, et de préférence de 5 % à 50 % en poids par rapport au poids total de la composition. Les huiles, les cires, les émulsionnants et les coémulsionnants utilisés dans la composition sous forme d'émulsion sont choisis parmi ceux classiquement utilisés dans le domaine cosmétique. L'émulsionnant et le coémulsionnant sont présents, dans la composition, en une proportion allant de 0,3 % à 30 % en poids, et de préférence de 0,5 à 20 % en poids par rapport au poids total de la composition. L'émulsion peut, en outre, contenir des vésicules lipidiques.

Lorsque la composition est une solution ou un gel huileux, la phase grasse peut représenter plus de 90 % du poids total de la composition.

De façon connue, la composition cosmétique peut contenir également des adjuvants habituels dans le domaine cosmétique, tels que les gélifiants hydrophiles ou lipophiles, les additifs hydrophiles ou lipophiles, les conservateurs, les antioxydants, les solvants, les parfums, les charges, les filtres, les absorbeurs d'odeur et les matières colorantes. Les quantités de ces différents adjuvants sont celles classiquement utilisées dans le domaine cosmétique, et par exemple de 0,01 % à 10 % du poids total de la composition. Ces adjuvants, selon leur nature, peuvent être introduits dans la phase grasse, dans la phase aqueuse et/ou dans les sphérules lipidiques.

Comme huiles ou cires utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles ou cires siliconées (cyclométhicone) et les huiles fluorées (perfluoropolyéthers), les cires d'abeille, de carnauba ou paraffine. On peut ajouter à ces huiles des alcools gras et des acides gras (acide stéarique).

Comme émulsionnants utilisables dans l'invention, on peut citer par exemple le stéarate de glycérol, le polysorbate 60 et le mélange de PEG-6/PEG-32/Glycol Stéarate vendu sous la dénomination de Tefose^{R} 63 par la société Gattefosse.

Comme solvants utilisables dans l'invention, on peut citer les alcools inférieurs, notamment l'éthanol et l'isopropanol, le propylène glycol.

Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium et la silice hydrophobe, éthylcellulose, polyéthylène.

La composition peut contenir d'autres actifs hydrophiles comme les protéines ou les hydrolysats de protéine, les acides aminés, les polyols, l'urée, l'allantoïne, les sucres et les dérivés de sucre, les vitamines hydrosolubles, les extraits végétaux et les hydroxyacides.

Comme actifs lipophiles, on peut utiliser le rétinol (vitamine A) et ses dérivés, le tocophérol (vitamine E) et ses dérivés, les acides gras essentiels, les céramides, les huiles essentielles, l'acide salicylique et ses dérivés.

Selon l'invention la composition peut associer au moins un extrait d'au moins un végétal de la famille des Rosacées à d'autres agents actifs destinés notamment à la prévention et/ou au traitement des affections cutanées. Parmi ces agents actifs, on peut citer à titre d'exemple :
- les agents modulant la différenciation et/ou la prolifération et/ou la pigmentation cutanée tels que l'acide rétinoïque et ses isomères, le rétinol et ses esters, la vitamine D et ses dérivés, les oestrogènes tels que l'oestradiol, l'acide kojique ou l'hydroquinone ;
- les antibactériens tels que le phosphate de clindamycine, l'érythromycine ou les antibiotiques de la classe des tétracyclines ;
- les antiparasitaires, en particulier le métronidazole, le crotamiton ou les pyréthrinoïdes ;
- les antifongiques, en particulier les composés appartenant à la classe des imidazoles tels que l'éconazole, le kétoconazole ou le miconazole ou leurs sels, les composés polyènes, tels que l'amphotéricine B, les composés de la famille des allylamines, tels que la terbinafine, ou encore l'octopirox ;
- les agents antiviraux tels que l'acyclovir ;
- les agents anti-inflammatoires stéroïdiens, tels que l'hydrocortisone, le valérate de bétaméthasone ou le propionate de clobétasol, ou les agents anti-inflammatoires non-stéroïdiens tels que l'ibuprofène et ses sels, le diclofénac et ses sels, l'acide acétylsalicylique, l'acétaminophène ou l'acide glycyrrhétinique ;
- les agents anesthésiques tels que le chlorhydrate de lidocaïne et ses dérivés ;
- les agents antiprurigineux comme la thénaldine, la triméprazine ou la cyproheptadine ;
- les agents kératolytiques tels que les acides alpha- et bêta-hydroxycarboxyliques ou bêta-cétocarboxyliques, leurs sels, amides ou esters et plus particulièrement les hydroxyacides tels que l'acide glycolique, l'acide lactique, l'acide salicylique, l'acide citrique et de manière générale les acides de fruits, et l'acide n-octanoyl-5-salicylique ;
- les agents anti-radicaux libres, tels que l'alpha-tocophérol ou ses esters, les superoxyde dismutases, certains chélatants de métaux ou l'acide ascorbique et ses esters ;
- les antiséborrhéiques tels que la progestérone ;
- les antipelliculaires comme l'octopirox ou la pyrithione de zinc ;
- les antiacnéiques comme l'acide rétinoïque ou le peroxyde de benzoyle ;
- les extraits végétaux ou d'origine microbienne.

Les exemples et compositions suivants illustrent l'invention sans la limiter aucunement. Dans les compositions les proportions indiquées sont des pourcentages en poids.

### Exemple 1 : Activité biologigue d'un extrait de Rosa gallica :

Mesure de l'affinité réceptorielle de l'extrait de pétales d'au moins un végétal de la famille des Rosacées pour le récepteur NK1 humain (récepteur à la substance P humain) :

1) La mesure de l'affinité réceptorielle de l'extrait de pétales d'au moins un végétal de la famille des Rosacées pour le récepteur NK1 humain a été effectuée selon la méthode décrite dans l'article : Heuillet, E. et al, J. Neurochem. ,60, 1993, 868-876.

L'extrait est testé aux concentrations de 1 %, 5 % et 10 % est un extrait de Rosa *gallica* vendu sous le nom d'Herbasol® par la société COSMETOCHEM's.

Lors de chaque expérience, la molécule de référence du récepteur étudié ([Sar⁹, Met (0₂)¹¹] SP, analogue de la substance P décrit par Heuillet, E. (Heuillet, E. et al, J. Neurochem. ,60, 1993, 868-876)) est parallèlement testée à 8 concentrations (n = 2) pour l'obtention d'une courbe standard permettant de valider l'expérimentation.

On obtient ainsi :
53 % de fixation pour l'extrait de l'exemple 1 à 1 %
90 % de fixation pour l'extrait de l'exemple 1 à 5 %
96 % de fixation pour l'extrait de l'exemple 1 à 10 %

Les résultats de cette expérience mettent en évidence une affinité de l'extrait pour le récepteur à la substance P humain dès la concentration de 1 %.

La courbe d'affinité tracée d'après les résultats obtenus montre 50 % de déplacement du ligand naturel (IC50) par l'extrait à la concentration de 2 %.

2) Un test fonctionnel *in vitro* réalisé sur le récepteur NK1 humain (récepteur à la substance P humain) présents sur les muscles lisses d'intestin isolé (iléon) est réalisé pour mettre en évidence le caractère antagoniste de substance P de l'extrait.

Les expériences *in vitro* sont réalisées selon la méthode décrite par Dion et al. (Life Sciences, 41, 1987, 2269-2278) et Patacchini et al. (Eur. J. Pharmacol., 215, 1992, 93-98).

Après installation dans des cuves expérimentales, les tissus (muscles lisses) sont soumis à une tension initiale de 1 g. Une période d'équilibration d'au moins 60 minutes, au cours de laquelle la solution physiologique est plusieurs fois renouvelée et la tension initiale réajustée, est ensuite respectée avant l'addition de l'extrait.

Les expériences sont conduites en présence continue d'atropine (3.10⁻⁶ M), de pyrilamine (3.10⁻⁶ M) et d'indométhacine (10⁻⁶ M) pour s'affranchir des effets indirects de médiateurs mis en jeu lors de la stimulation d'autres types de récepteurs présents sur ce tissu.

Chaque préparation est initialement stimulée par un agoniste de substance P : [Sar⁹, Met (0₂)¹¹ SP], à la concentration de 10⁻⁸ M pour l'obtention d'une réponse contractile "contrôle", puis la solution physiologique est entièrement renouvelée.

Cette opération est ensuite répétée toutes les 40 minutes en présence de concentrations croissantes de l'extrait de pétales d'au moins un végétal de la famille des Rosacées, chacune d'elles étant ajoutée 30 minutes avant la [Sar⁹, Met (0₂)¹¹ SP].

Une inhibition de 50 % de l'activité de la [Sar⁹, Met (0₂)¹¹ SP] est obtenue à la concentration de 1% en extrait.

### Conclusions :

L'extrait présente une affinité pour le récepteur de la substance P et exerce une activité spécifique antagoniste de la substance P.

### Exemple 2 :

Exemples de formulations illustrant l'invention et particulièrement les compositions selon l'invention associant au moins un extrait de pétales d'au moins un végétal de la famille des Rosacées et un produit à effet irritant. Ces compositions ont été obtenues par simple mélange des différents composants.

| Composition 1 : Lotion démaquillante pour le visage | |
|---|---|
| Herbasol® | 1,00 |
| Antioxydant | 0,05 |
| Isopropanol | 40,00 |
| Conservateur | 0,30 |
| Eau | qsp 100 % |
| | |

| Composition 2 : Gel pour le soin du visage | |
|---|---|
| Herbasol® | 4,00 |
| Hydroxypropylcellulose* | 1,00 |
| Antioxydant | 0,05 |
| Isopropanol | 40,00 |
| Conservateur | 0,30 |
| Eau | qsp 100 % |
| | |

| Composition 3 : Crème de soin du visage (émulsion huile dans eau) | |
|---|---|
| Herbasol® | 5,00 |
| Stéarate de glycérol | 2,00 |
| Polysorbate 60** | 1,00 |
| Acide stéarique | 1,40 |
| Triéthanolamine | 0,70 |
| Carbomer | 0,40 |
| Fraction liquide du beurre de karité | 12,00 |
| Perhydrosqualène | 12,00 |
| Antioxydant | 0,05 |
| Parfum | 0,5 |
| Conservateur | 0,30 |
| Eau | qsp 100 % |
| | |

| Composition 4 : Shampooing | |
|---|---|
| Herbasol® | 2,00 |
| Hydroxypropylcellulose* | 1,00 |
| Parfum | 0,50 |
| Conservateur | 0,30 |
| Eau | qsp 100 % |
| | |

| Composition 5 : Crème de soin antirides pour le visage (émulsion huile/eau) | |
|---|---|
| Herbasol® | 6,00 |
| Stéarate de glycérol | 2,00 |
| Polysorbate 60** | 1,00 |
| Acide stéarique | 1,40 |
| Acide n-octanoyl-5-salicylique | 0,50 |
| Triéthanolamine | 0,70 |
| Carbomer | 0,40 |
| Fraction liquide du beurre de karité | 12,00 |
| Perhydrosqualène | 12,00 |
| Antioxydant | 0,05 |
| Parfum | 0,5 |
| Conservateur | 0,30 |
| Eau | qsp 100 % |
| | |

| Composition 6 : Gel anti-douleur | |
|---|---|
| Herbasol® | 10,00 |
| Hydroxypropylcellulose* | 1,00 |
| Antioxydant | 0,05 |
| Chlorhydrate de lidocaïne | 2,00 |
| Isopropanol | 40,00 |
| Conservateur | 0,30 |
| Eau | qsp 100 % |
| | |

| Composition 7 : Crème de soin de l'érythème solaire (émulsion huile-dans-eau) | |
|---|---|
| Herbasol® | 2,00 |
| Stéarate de glycérol | 2,00 |
| Polysorbate 60** | 1,00 |
| Acide stéarique | 1,40 |
| Acide glycyrrhétinique | 2,00 |
| Triéthanolamine | 0,70 |
| Carbomer | 0,40 |
| Fraction liquide du beurre de karité | 12,00 |
| Huile de tournesol | 10,00 |
| Antioxydant | 0,05 |
| Parfum | 0,5 |
| Conservateur | 0,30 |
| Eau | qsp 100 % |
| | |

| Composition 8 : Gel pour le traitement de l'acné | |
|---|---|
| Herbasol® | 8,00 |
| Acide tout trans rétinoïque | 0,05 |
| Hydroxypropylcellulose* | 1,00 |
| Antioxydant | 0,05 |
| Isopropanol | 40,00 |
| Conservateur | 0,30 |
| Eau | qsp 100 % |
| | |

| Composition 9 : Lotion pour éliminer les cicatrices dues à l'acné | |
|---|---|
| Herbasol® | 2,50 |
| Acide glycolique | 50,00 |
| Hydroxypropylcellulose* | 0,05 |
| NaOH | qsp pH = 2,8 |
| Ethanol | qsp 100 % |
| Conservateur | 0,30 |

| | |
|---|---|
| * : Klucel H® vendu par la société Hercules | |
| ** : Tween 60® vendu par la société ICI | |

## Revendications

1. Composition **caractérisée en ce qu'**elle comprend, dans un milieu physiologiquement acceptable, au moins un extrait d'au moins un végétal de la famille des Rosacées appartenant au genre Rosa et au moins un composé diminuant la synthèse, la libération et/ou l'activité d'au moins un médiateur de l'inflammation.

2. Composition selon la revendication 1, **caractérisée par le fait que** le composé diminuant la synthèse, la libération et/ou l'activité d'au moins un médiateur de l'inflammation est de préférence choisi parmi les antagonistes de substance P et/ou de CGRP, les inhibiteurs de NO-synthase, les antagonistes de bradykinine, les antagonistes de cytokines, les antagonistes d'histamine, les antagonistes du facteur de nécrose tumorale de type α (TNFα).

3. Composition selon la revendication 2, **caractérisée par le fait que** l'antagoniste de substance P est choisi parmi les substances assurant une diminution de l'extravasation du plasma au travers de la paroi vasculaire induite par la capsaïcine ou par une stimulation nerveuse antidromique et les substances provoquant une inhibition de la contraction des muscles lisses induites par l'administration de substance P.

4. Composition selon la revendication 3, **caractérisée par le fait que** l'antagoniste de substance P est choisi parmi les peptides, les composés non peptidiques comprenant au moins un hétérocycle, les composés azotés comprenant au moins un cycle benzénique, les sels de cations monovalents, divalents et trivalents, les eaux thermales, les extraits végétaux et les extraits de microorganismes.

5. Composition selon la revendication 4, **caractérisée par le fait que** l'antagoniste de substance P est un sel de cations choisi parmi les sels de strontium, de magnésium, de lanthanides de numéro atomique allant de 57 à 71, de cobalt, de nickel, de manganèse, de baryum, d'yttrium, de cuivre, d'étain, de rubidium, de lithium et de zinc.

6. Composition selon la revendication 4, **caractérisée par le fait que** l'antagoniste de substance P est une eau thermale choisie parmi les eaux thermales du bassin de Vichy, comme celles provenant des sources Célestins, Chomel, Grande-Grille, Hôpital, Lucas et Parc.

7. Composition selon la revendication 6, **caractérisée par le fait que** l' eau thermale du bassin de Vichy est choisie parmi celles provenant des sources Célestins, Chomel, Grande-Grille, Hôpital, Lucas et Parc.

8. Composition selon la revendication 2, **caractérisée par le fait que** l'antagoniste de CGRP est choisi parmi :
+ la substance antagoniste diminuant la vasodilatation induite par la capsaïcine et/ou par une stimulation électrique antidromique (appliquée sur un nerf afférent) et/ou
+ la substance antagoniste provoquant une inhibition de la libération de CGRP par les fibres nerveuses sensitives et/ou
+ la substance antagoniste provoquant une inhibition de la contraction du muscle lisse du vas deferens induite par le CGRP.

9. Composition selon la revendication 8, **caractérisée par le fait que** l'antagoniste de CGRP est choisi parmi CGRP 8-37, les anticorps anti-CGRP ou les extraits végétaux préparés à partir de végétaux de la famille des Iridacées.

10. Composition selon la revendication 2, **caractérisée par le fait que** les inhibiteurs de NO-synthase sont choisis parmi les composés inhibant la synthèse et/ou accélérant le catabolisme de la NO-synthase, les composés neutralisant la NO-synthase ou les composés intervenant en diminuant le signal transduit par la NO-synthase.

11. Composition selon la revendication 10, **caractérisée par le fait que** les inhibteurs de NO-synthase sont choisis parmi la N^{G}-monométhyl-L-arginine (L-NMMA), la N^{G}-nitro-L-arginine, l'ester méthylé de la N^{G}-nitro-L-arginine, le chlorure de diphénylèneiodonium, la 7-nitroindazole, la N(5)-(1-iminoéthyl)-L-ornithine, la N^{G},N^{G}-diméthyl-L-arginine, la N^{G},N^{G}-diméthyl-arginine, le 2-(4-carboxyphényl)-4,4,5,5-tetraméthylimidazoline-1-oxy-3-oxyde, l'aminoguanidine, la canavanine, l'ebselen et l'hormone stimulatrice des mélanocytes de type α.

12. Composition selon la revendication 2, **caractérisée par le fait que** les antagonistes de la bradykinine sont choisis parmi des composés inhibant la synthèse et/ou accélérant le catabolisme de la bradykinine, des composés neutralisant la bradykinine, des composés bloquant les récepteurs de la bradykinine tels que ceux qui interférent avec les effets de la bradykinine par leur fixation au récepteur de celle-ci (B1 ou B2), des composés inhibant la synthèse des récepteurs de la bradykinine ou des composés intervenant en diminuant le signal transduit par la bradykinine.

13. Composition selon la revendication 12, **caractérisée par le fait que** les antagonistes de bradykinine sont choisis parmi la D-Arg, [Hyp3, D-Phe7]-bradykinin (NPC567), la [Thi 5, 8, D-Phe7]-bradykinin, la D-Arg, [Hyp3, Thi5,8, D-Phe7]-bradykinin, la N-α-adamantaneacetyl-D-Arg, [Hyp3, Thi5,8, D-Phe7]-bradykinin, la des-Arg9, [Leu8]-bradykinin, la P-guanidobenzoyl, [Hyp3,Thi5,D-Tic7,Oic8]-bradyklnin (S 16118), la D-Arg, [Hyp3, Thi5, D-Tic7,Oic8]-bradykinine (HOE 140), la D-Arg, [Hyp3, D-Hype (trans-propyl) 7, Oic 8]-bradykinin (NPC 17731).

14. Composition selon la revendication 2, **caractérisée par le fait que** les antagonistes d'histamine, de cytokines et/ou de TNF-α sont choisis parmi les substances susceptibles d'inhiber la libération et/ou la synthèse et/ou la fixation réceptorielle respectivement d'histamine, de cytokines et/ou de TNF-α.

15. Composition selon l'une quelconque des revendications 1 à 14, **caractérisée par le fait que** le composé diminuant la synthèse, la libération et/ou l'activité d'au moins un médiateur de l'inflammation en une quantité représentant de 0,001% à 10% du poids total de la composition et préférentiellement en une quantité représentant de 0,01% à 5% du poids total de la composition.

16. Composition selon l'une quelconque des revendications 1 à 15, **caractérisée par le fait qu'**elle est à usage cosmétique ou pharmaceutique.

17. Composition selon l'une quelconque des revendications 1 à 16, **caractérisée par le fait que** l'extrait d'au moins un végétal de la famille des Rosacées est obtenu à partir de matériel végétal issu de plante entière ou de feuilles, de tiges, de fleurs, de pétales, de racines ou encore de cellules indifférenciées.

18. Composition selon la revendication 17, **caractérisée par le fait que** l'extrait d'au moins un végétal de la famille des Rosacées est obtenu à partir de pétales.

19. Composition selon l'une quelconque des revendications 1 à 18, **caractérisée par le fait que** l'extrait d'au moins un végétal de la famille des Rosacées est obtenu à partir de matériel végétal issu d'au moins un végétal de la famille des Rosacées cultivé *in vivo*.

20. Composition selon l'une quelconque des revendications 1 à 19, **caractérisée par le fait que** l'extrait d'au moins un végétal de la famille des Rosacées est en une quantité représentant de 0,01% à 30% du poids total de la composition et préférentiellement en une quantité représentant de 0,1% à 20% du poids total de la composition.

21. Composition selon l'une quelconque des revendications 1 à 20, **caractérisée par le fait que** l'extrait est un extrait préparé à partir de matériel issu d'au moins un végétal appartenant à une espèce choisie parmi *Rosa alba, Rosa alpina, Rosa canina, Rosa cinnamonea, Rosa gallica, Rosa repens, Rosa rubrifolia, Rosa rubiginosa, Rosa sempervirens, Rosa spinosissima, Rosa stylosa, Rosa tomentosa, Rosa villosa*.

22. Composition selon l'une quelconque des revendications 1 à 21, **caractérisée par le fait que** le végétal appartient à l'espèce Rosa *gallica*.
